# EUROPEAN PATENT SPECIFICATION

(11) **EP 1 320 589 B1**
(45) Date of publication and mention of the grant of the patent: **08.10.2008**
(21) Application number: 01979922.0
(22) Date of filing: 27.09.2001
(51) Int. Cl.: C12N 9/68, C12N 9/74, A61K 38/48

(54) **ANTIANGIOGENIC POLYPEPTIDES AND METHODS FOR INHIBITING ANGIOGENESIS**
ANTIANGIOGENISCHE POLYPEPTIDE UND VERFAHREN ZUR ANGIOGENESEHEMMUNG
POLYPEPTIDES ANTIANGIOGENIQUES ET METHODES D'INHIBITION DE L'ANGIOGENESE

(30) Priority: 29.09.2000 US 675226; 31.08.2001 US 942704
(43) Date of publication of application: 25.06.2003
(73) Proprietor: ABBOTT LABORATORIES, Abbott Park IL 60064-3500 (US)
(72) Inventor: HENKIN, Jack, Highland Park, IL 60035 (US); DAVIDSON, Donald J., Gurnee, IL 60031 (US)
(74) Representative: Modiano, Micaela Nadia
(86) International application number: PCT/US2001/042423
(87) International publication number: WO 2002/026782

(56) References cited:
- WO-A-00/14209
- WO-A-96/35774
- WO-A-96/41813
- WO-A-97/41824

## Description

### Cross-Reference to Related Applications

This application claims priority to U.S. Provisional Patent Application Ser. No. 60/236,550, filed September 29, 2000, which is hereby incorporated by reference.

### Technical Field

The present invention relates to the field of peptide chemistry. More particularly, the invention relates to the preparation and use of conjugated peptides containing amino acid sequences substantially similar to the corresponding sequences of mammalian plasminogen, pharmaceutical compositions containing the peptides, and treatment of diseases which arise from or are exacerbated by angiogenesis.

### Background of the Invention

Angiogenesis, the process by which new blood vessels are formed, is essential for normal body activities including reproduction, development and wound repair. Although the process is not completely understood, it is believed to involve a complex interplay of molecules which regulate the growth of endothelial cells (the primary cells of capillary blood vessels). Under normal conditions, these molecules appear to maintain the microvasculature in a quiescent state (i.e. one of no capillary growth) for prolonged periods which may last for as long as weeks or, in some cases, decades. When necessary (such as during wound repair), these same cells can undergo rapid proliferation and turnover within a 5 day period (Folkman, J. and Shing, Y., The Journal of Biological Chemistry, 267(16), 10931-10934, and Folkman, J. and Klagsbrun, M., Science, 235, 442-447 (1987)).

Although angiogenesis is a highly regulated process under normal conditions, many diseases (characterized as angiogenic diseases) are driven by persistent unregulated angiogenesis. Otherwise stated, unregulated angiogenesis may either cause a particular disease directly or exacerbate an existing pathological condition. For example, ocular neovascularization has been implicated as the most common cause of blindness and dominates approximately 20 eye diseases. In certain existing conditions, such as arthritis, newly formed capillary blood vessels invade the joints and destroy cartilage. In diabetes, new capillaries formed in the retina invade the vitreous, bleed, and cause blindness. Growth and metastasis of solid tumors are also dependent on angiogenesis (Folkman, J., Cancer Research, 46, 467-473 (1986), Folkman, J., Journal of the National Cancer Institute, 82, 4-6 (1989)). It has been shown, for example, that tumors which enlarge to greater than 2 mm must obtain their own blood supply and do so by inducing the growth of new capillary blood vessels. Once these new blood vessels become embedded in the tumor, they provide a means for tumor cells to enter the circulation and metastasize to distant sites such as liver, lung or bone (Weidner, N., et al, The New England Journal of Medicine, 324(1): 1-8 (1991)).

Several angiogenesis inhibitors are currently under development for use in treating angiogenic diseases, but there are disadvantages associated with these compounds. Fumagillin, a compound secreted by the fungus *Aspergillus fumigatis fresenius,* has demonstrated angioinhibitory effects, but has not been developed clinically due to the dramatic weight loss suffered by laboratory animals after prolonged exposure. TNP-470, a synthetic analog of fumagillin, also inhibits endothelial growth but has been shown to induce asthenial and neurocortical toxicity in humans**,** limiting allowable dosages (J Clin. Oncology 17,2541(1989)).

To date, several angiogenic factors, specifically kringle peptide fragments of plasminogen have been described (see, for example, US Patent No. 5,639,725; US Patent No. 5,733,876; US Patent No. 5,792,943; US Patent No. 5,837,682; US Patent No. 5,861,372; US Patent No. 5,885,795; US Patent No. 6,024,688; US Patent No. 5,854,221, US Patent No. 5,801,146; US Patent No. 5,981,484; US Patent No. 6,057,122; and US Patent No. 5,972,896, which are incorporated herein by reference in their entirety). Despite the promising anti-angiogenic activity of these fragments, their circulation half-lives are short. Thus, there is still a need for anti-angiogenic compounds, such as kringle peptide fragments, which have improved pharmacokinetic activity. Such compounds should also be easily and cost-effectively made.

### Summary of the Invention

In its principle embodiment, the present invention discloses a conjugated kringle peptide fragment selected from the group consisting of mPSG-SLLPD-K5 and mPEG-SEED-K5.

In another embodiment, the present invention discloses a pharmaceutical composition comprising a conjugated kringle peptide fragment selected from the group consisting of mPEG-SLLPD-K5 and mPEG-SEED-K5 in combination with a therapeutically acceptable carrier.

In another embodiment, the present invention discloses a method of treating a disease in a patient in need of anti-angiogenic therapy comprising administering to a human or animal a therapeutically effective amount of a conjugated kringle peptide fragment selected from the group consisting of mPEG-SLLPD-K5 and mPEG-SEED-K5. Preferably, the disease is selected from the group consisting of cancer, arthritis, macular degeneration, and diabetic retinopathy. More preferably, the disease is cancer, and most preferably, the disease is selected from the group consisting of primary and metastatic solid tumors, carcinomas, sarcomas, lymphomas, psoriasis and hemagiomas.

In another embodiment, the present invention discloses a method of inhibiting endothelial cell proliferation in an individual comprising administering to said individual an effective amount of a conjugated kringle peptide fragment selected from the group consisting of mPEG-SLLPD-K5 and m-PEG-SEED-K5.

### Detailed Description of the Invention

As used herein, the singular forms "a", "an", and "the" include plural reference unless the context clearly dictates otherwise. In addition, all published scientific and patent literature (including patent applications) referred to herein are hereby incorporated by reference in their entirety.

As used herein, the terms "K5" ,"Kringle 5","K1", and "Kringle 1" refer to kringle regions of plasminogen.

As used herein, the term "K2" refers to a kringle region of prothrombin.

Unless otherwise stated, the following terms shall have the following meanings:

The term "acyl group" means a C₁-C₁₀ alkyl group attached to the parent molecular moiety through a carbonyl group.

The term "acylating group" means a reagent capable of donating an acyl group to the nitrogen atom of a molecule during the course of a reaction. Examples of acylating groups include acetic anhydride, acetyl chloride, and the like.

The term "C₁-C₁₀ alkyl" means a monovalent group of one to ten carbon atoms derived from a straight or branched chain saturated hydrocarbon by the removal of a single hydrogen atom.

The term "C₁-C₁₀ alkylene" means a divalent group of one to ten carbon atoms derived from a straight or branched chain saturated hydrocarbon.

The term "carbonyl group" means -C(O)-.

The term "conjugate of a kringle peptide fragment" or "conjugated kringle peptide fragment" means a functionalized kringle peptide fragment chemically coupled to a functionalized polymer. Methods of making such conjugates have been described (see, for example, WO96/41813 and J. Pharmaceut. Sci. 87, 1446-1449 (1998)). Non-limiting examples of conjugates of kringle peptide fragments include a kringle 5 peptide fragment coupled to polyethylene glycol, a kringle 5 peptide fragment coupled to methoxypolyethylene glycol, a kringle 4-5 peptide coupled to methoxypolyethylene glycol, and the like.

The term "functionalized kringle peptide fragment" refers to a kringle peptide fragment, as defined herein, which has been modified site-specifically to contain a reactive group. Functionalized kringle peptide fragments also are intended to include kringle peptide fragments which have been modified by the addition of one or more non-native amino acids at the N-terminus. The additional amino acid may serve as the reactive group or, alternatively, may itself be further modified to form a reactive group. For example, a serine (Ser) may be added immediately upstream (i.e., before) the leucine (Leu) residue at position 450 of a kringle 5 peptide fragment having the sequence from amino acid position 450 to amino acid position 543. The N-terminal Ser residue may then be specifically modified to form a reactive group such as an aldehyde. Kringle peptide fragments having one or more non-native amino acids at their N-terminus can be made by any means well known in the art, such as, for example, by recombinant DNA methodologies or by synthetic peptide chemistry. Means for functionalizing polypeptides are known to those of ordinary skill in the art (see, for example, WO96/41813 and J. Pharmaceut. Sci. 87, 1446-1449 (1998)).

The term "functional or functionalized polymer" means the formation of either an amino-oxy group or an aldehyde on a polymer, as defined herein, so that the polymer can be site-specifically conjugated to a complementary functionalized target, such as a functionalized kringle peptide fragment. By way of example, means to functionalize polymers also are provided in WO96/41813 and J. Pharmaceut. Sci. 87, 1446-1449 (1998)).

The term "kringle fusion protein" refers to a polypeptide comprising an amino acid sequence drawn from two or more individual polypeptides, one of which is a kringle peptide fragment. A kringle fusion protein may be formed by the expression of a polynucleotide in which the coding sequence for a kringle peptide fragment has been joined with the coding sequence of at least one other polypeptide such that the two (or more) reading frames are in frame.

The term "kringle peptide fragment" refers to a kringle region of plasminogen having anti-angiogenic activity and includes a sequence of: (a) an individual kringle peptide such as kringle 5 (K5) of plasminogen; (b) a kringle peptide having substantial sequence homology to the sequences described in (a) and (c) a peptide segment of (a), which has substantial sequence homology to the sequence described in (a), The aforementioned kringle peptide fragments and methods for making and using said fragments have been extensively described in the patent literature (see U.S. Patent No. 5,639,725; U.S. Patent No. 5,733,876; U.S. Patent No. 5,792,845; U.S. Patent No. 5,837,682; U.S. Patent No. 5,861,372; U.S. Patent No. 5,885,795; U. S. Patent No. 6,024,688; U. S. Patent No. 5, 854,221, U.S. Patent No. 5, 801,146; U. S. Patent No. 5,981,484; U.S. Patent No. 6,057,122; and U.S. Patent No. 5,972,896) as well as in the scientific literature.

Particularly preferred K5 peptide fragments have the sequences from amino acid residue 450 to 543 of SEQ ID NO:1 and from amino acid residue 458-543 of SEQ m NO:1.

The term "polymer" means a chemical compound consisting of repeating non-peptide structural units. Preferred polymer of the present invention is methoxypolyethylene glycol (mPEG).

The terms "polypeptide", "peptide", and "protein" each refer to a molecular chain of amino acids and does not refer to a specific chain length. This term is also intended to refer to post-expression modifications of the polypeptides, peptides, and proteins, for example, glycosylations, acetylations, phosphorylations and the like.

The term "purified polypeptide" means a polypeptide of interest or fragment thereof which is essentially free, that is, contain less than about 50%; preferably less than about 70%, and more preferably, less than about 90% of cellular components with which the polypeptide of interest is naturally associated. Methods for purifying polypeptides are well known in the art.

The term "substantial sequence homology" means approximately 60% amino acid identity, desirably at least approximately 70% amino acid identity, more desirably approximately 80% amino acid identity, even more desirably approximately 90% identity and most desirably approximately 95% amino acid identity of the corresponding peptide sequence of human, murine, bovine, canine, feline, Rhesus monkey, or monkey plasminogen. Sequences having substantial sequence homology to human plasminogen are referred to as "homologues". In addition to having substantial sequence homology, homologues of the present invention demonstrate like biological activity (i.e., anti-angiogenic activity) as kringle peptide fragments described herein. Because the amino acid sequence or the number of amino acids in a kringle peptide fragment may vary from species to species or from the method of production, the total number of amino acids in a kringle peptide fragment cannot, in some instances, be defined exactly. Given that these sequences are identical in at least 73% of their amino acids, if is to be understood, that the amino acid sequence of a kringle peptide fragment is substantially similar among species and that methods of production of kringle peptide fragments provide kringle peptide fragments with substantial sequence homology to the corresponding amino acid sequences of human plasminogen

All peptide sequences are written according to the generally accepted convention whereby the α-N-terminal amino acid residue is on the left and the α-C-terminal is on the right. As used herein, the term "α-N-terminal" refers to the free alpha-amino group of an amino acid in a peptide, and the term "α-C-terminal" refers to the free alpha-carboxylic acid terminus of an amino acid in a peptide.

The present invention provides compounds having anti-angiogenic activity comprising a functionalized kringle peptide fragment conjugated to a functionalized polymer selected from the group consisting of mPEG-SLLPD-K5 and inPEG-SEED-K5. The formation of such conjugates renders them more suitable as therapeutic agents. For example, desirable properties of conjugated kringle peptide fragments include increased solubility in aqueous solutions, increased stability during storage, reduced immunogenicity, increased resistance to enzymatic degradation, compatibility with a wider variety of drug administration systems, and increased *in vivo* half-lives

The invention also provides methods for inhibiting endothelial cell proliferation using a conjugated kringle peptide fragment selected from the group consisting of mPEG-SLLPD-K5 and mPEG-SEED-K5 as well as methods for treating a human or animal in need of anti-angiogenic therapy comprising administering to such individuals a therapeutically effective amount of said conjugate of a kringle peptide fragment.

The invention further provides pharmaceutical compositions comprising a conjugate of a kringle peptide fragment selected from the group consisting of mPEG-SLLPD-K5 and mPEG-SEED-KS, and a pharmaceutically acceptable excipient.

The compounds of the invention, possess anti-angiogenic activity. As angiogenesis inhibitors, such compounds are useful in the treatment of both primary and metastatic solid tumors and carcinomas of the breast; colon; rectum; lung; oropharynx; hypopharynx; esophagus; stomach; pancreas; liver; gallbladder, bile ducts; small intestine; urinary tract including kidney, bladder and urothelium; female genital tract including cervix, uterus, ovaries, choriocarcinoma and gestational trophoblastic disease; male genital tract including prostate, seminal vesicles, testes and germ cell tumors; endocrine glands including thyroid, adrenal, and pituitary; skin including hemangiomas, melanomas, sarcomas arising from bone or soft tissues and Kaposi's sarcoma; tumors of the brain, nerves, eyes, and meninges including astrocytomas, gliomas, glioblastomas, retinoblastomas, neuromas, neuroblastomas, Schwannomas and meningiomas; solid tumors arising from hematopoietic malignancies such as leukemia and including chloromas, plasmacytomas, plaques and tumors of mycosis fimgoides and cutaneous T-cell lymphoma/leukemia; lymphomas including both Hodgkin's and non-Hodgkin's lymphomas; prophylaxis of autoimmune diseases including rheumatoid, immune and degenerative arthritis; ocular diseases including diabetic retinopathy, retinopathy of prematurity, corneal graft rejection, retrolental fibroplasia, neovascular glaucoma, rubeosis, retinal neovascularization due to mater degeneration and hypoxia; abnormal neovascularization conditions of the eye; skin diseases including psoriasis; blood vessel diseases including hemagiomas and capillary proliferation within atherosclerotic plaques; Osler-Webber Syndrome; myocardial angiogenesis; plaque neovascularization; telangiectasia; hemophiliac joints; angiofibroma; wound granulation; diseases characterized by excessive or abnormal stimulation of endothelial cells including intestinal adhesions, Crohn's disease, atherosclerosis, scleroderma and hypertrophic scars (i.e. keloids) and diseases which have angiogenesis as a pathologic consequence including cat scratch disease (*Rochele minalia quintosa*) and ulcers (*Helicobacter pylori*)*.* Another use is as a birth control agent which inhibits ovulation and establishment of the placenta.

The compounds of the present invention may also be useful for the prevention of metastases from the tumors described above either when used alone or in combination with radiotherapy and/or other chemotherapeutic treatments conventionally administered to patients for treating angiogenic diseases and/or in combination with other anti-angiogenic agents. For example, when used in the treatment of solid tumors, compounds of the present invention may be administered with chemotherapeutic agents such as alpha interferon, COMP (cyclophosphamide, vincristine, methotrexate and prednisone), etoposide, mBACOD (methortrexate, bleomycin, doxorubicin, cyclophosphamide, vincristine and dexamethasone), PRO-MACE/MOPP (prednisone, methotrexate (w/leucovin rescue), doxorubicin, cyclophosphamide, taxol, etoposide/mechlorethamine, vincristine, prednisone and procarbazine), vincristine, vinblastine, angioinhibins, TNP-470, pentosan polysulfate, platelet factor 4, angiostatin, LM-609, SU-101, CM-101, Techgalan, thalidomide, SP-PG and the like. Other chemotherapeutic agents include alkylating agents such as nitrogen mustards including mechloethamine, melphan, chlorambucil, cyclophosphamide and ifosfamide; nitrosoureas including carmustine, lomustine, semustine and streptozocin; alkyl sulfonates including busulfan; triazines including dacarbazine; ethyenimines including thiotepa and hexamethylmelamine; folic acid analogs including methotrexate; pyrimidine analogues including 5-fluorouracil, cytosine arabinoside; purine analogs including 6-mercaptopurine and 6-thioguanine; anti-tumor antibiotics including actinomycin D; the anthracyclines including doxorubicin, bleomycin, mitomycin C and methramycin; hormones and hormone antagonists including tamoxifen and cortiosteroids and miscellaneous agents including cisplatin and brequinar. For example, a tumor may be treated conventionally with surgery, radiation or chemotherapy and conjugated kringle 5 administration with subsequent conjugated kringle 5 administration to extend the dormancy of micrometastases and to stabilize and inhibit the growth of any residual primary tumor.

The term "parenteral," as used herein, refers to modes of administration which include intravenous, intramuscular, intraperitoneal, intrasternal, subcutaneous and intraarticular injection and infusion. Pharmaceutical compositions for parenteral injection comprise pharmaceutically acceptable sterile aqueous or non-aqueous solutions, dispersions, suspensions or emulsions as well as sterile powders for reconstitution into sterile injectable solutions or dispersions just prior to use. Examples of suitable aqueous and non-aqueous carriers, diluents, solvents or vehicles include water, ethanol, polyols (such as glycerol, propylene glycol, polyethylene glycol and the like), carboxymethylcellulose and suitable mixtures thereof, vegetable oils (such as olive oil) and injectable organic esters such as ethyl oleate. Proper fluidity may be maintained, for example, by the use of coating materials such as lecithin, by the maintenance of the required particle size in the case of dispersions and by the use of surfactants. These compositions may also contain adjuvants such as preservatives, wetting agents, emulsifying agents and dispersing agents. Prevention of the action of microorganisms may be ensured by the inclusion of various antibacterial and antifungal agents such as paraben, chlorobutanol, phenol sorbic acid and the like. It may also be desirable to include isotonic agents such as sugars, sodium chloride and the like. Prolonged absorption of the injectable pharmaceutical form may be brought about by the inclusion of agents, such as aluminum monostearate and gelatin, which delay absorption. Injectable depot forms are made by forming microencapsule matrices of the drug in biodegradable polymers such as polylactide-polyglycolide, poly(orthoesters) and poly(anhydrides). Depending upon the ratio of drug to polymer and the nature of the particular polymer employed, the rate of drug release can be controlled. Depot injectable formulations are also prepared by entrapping the drug in liposomes or microemulsions which are compatible with body tissues. The injectable formulations may be sterilized, for example, by filtration through a bacterial-retaining filter or by incorporating sterilizing agents in the form of sterile solid compositions which can be dissolved or dispersed in sterile water or other sterile injectable media just prior to use.

Topical administration includes administration to the skin, mucosa and surfaces of the lung. Compositions for topical administration may be prepared as a dry powder which may be pressurized or non-pressurized. In non-pressurized powder compositions, the active ingredient in finely divided form may be used in admixture with a larger-sized pharmaceutically acceptable inert carrier comprising particles having a size, for example, of up to 100 micrometers in diameter. Suitable inert carriers include sugars such as lactose. Desirably, at least 95% by weight of the particles of the active ingredient have an effective particle size in the range of 0.01 to 10 micrometers. Alternatively, a compound of the invention may be injected directly into the vitreous and aqueous humor of the eye.

Compounds of the present invention may also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes can be used. The present compositions in liposome form may contain, in addition to a compound of the present invention, stabilizers, preservatives, excipients and the like. The preferred lipids are the phospholipids and the phosphatidyl cholines (lecithins), both natural and synthetic. Methods to form liposomes are known in the art.

When used in the above or other treatments, a therapeutically effective amount of one of the compounds of the present invention may be employed in pure form or, where such forms exist, in pharmaceutically acceptable salt form and with or without a pharmaceutically acceptable excipient. A "therapeutically effective amount" of the compound of the invention means a sufficient amount of the compound to treat an angiogenic disease (for example, to limit tumor growth or to slow or block tumor metastasis) at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed; the age, body weight, general health, sex and diet of the patient; the time of administration; the route of administration; the rate of excretion of the specific compound employed; the duration of the treatment; drugs used in combination or coincidental with the specific compound employed and like factors well known in the medical arts. For example, it is well within the skill of the art to start doses of the compound at levels lower than those required to achieve the desired therapeutic effect and to gradually increase the dosage until the desired effect is achieved. Total daily dose of conjugated kringle 5 peptide fragments to be administered locally or systemically to a human or other mammal host in single or divided doses may be in amounts, for example, from 0.01 to 5 mg/kg body weight daily and more usually 0.05 to 1 mg/kg body weight. If desired, the effective daily dose may be divided into multiple doses for purposes of administration. Consequently, single dose compositions may contain such amounts or submultiples thereof to make up the daily dose.

It will be understood that agents which can be combined with the compound of the present invention for the inhibition, treatment or prophylaxis of angiogenic diseases are not limited to those listed above, but include, in principle, any agents useful for the treatment or prophylaxis of angiogenic diseases.

### Synthetic Processes

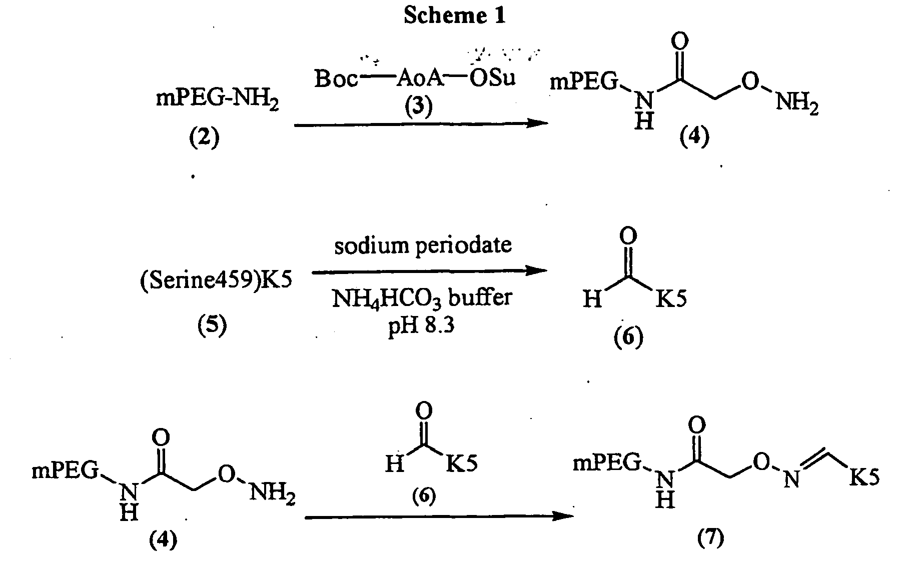

Scheme 1 shows the preparation of one type of polyalkylene glycol conjugate of K5, which can readily be extended to the synthesis of other conjugated kringle peptide fragments. Aminomethoxypolyethylene glycols (**2**) (5-30kD) can be condensed with (N-tert-butoxycarbonylamino)oxy)acetyl N-hydroxysuccinimide ester (**3**) to provide (amino-oxy)acetyl-functionalized polyethylene glycols (**4**). These can be condensed with aldehyde (**6**) (formed by the oxidation of (Serine459)K5 (**5**) with sodium periodate) to form the desired conjugated peptide fragments (**7**) with varying polyethylene glycol lengths.

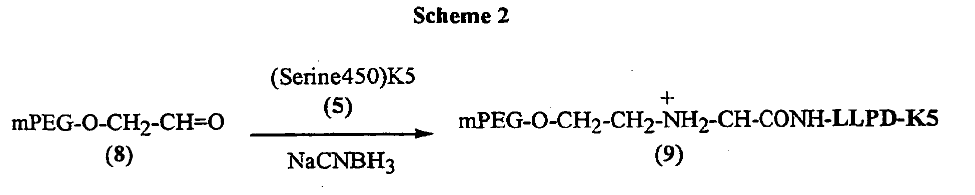

Scheme 2 shows the preparation of an alternate polyethylene glycol conjugate of K5, which can also readily be extended to the synthesis of other conjugated kringle peptide fragments. Methoxypolyethylene glycol aldehyde (**8**) (5-30kD) can be condensed with (Serine459)K5 in the presence of a reducing agent such as sodium cyanoborohydride to form the desired conjugated peptide fragments (**9**) (LLPD-K5 = leucine-leucine-proline-aspartic acid-K5) with varying polyethylene glycol lengths.

The following examples will serve to further illustrate the preparation of the novel compounds of the invention:

### Example 1

### Construction of pET32-Kan-Ek-SLLPD-K5, pET32-Kan-Ek-SEED-K5, pET32-Kan-Tev-SEED-K5

PCR was employed to generate Kringle fusion proteins which comprise a kringle 5 peptide fragment. A 5' PCR primer coding for an Enterokinase cleavage site Asp-Asp-Asp-Asp-Lys (**SEQ ID NO:2**) followed by a Ser residue and the K5 N-terminal coding sequences beginning at plasminogen residue 450 and extending to residue 453 of SEQ ID NO:1 was synthesized: TGGGTACCGACGACGACGACAAGTCCCTGCTTCCAGATGTAGAGA (**SEQ ID NO:3**). A 5' PCR primer coding for an Enterokinase cleavage site Asp-Asp-Asp-Asp-Lys (**SEQ ID NO:2**) followed by the K5 N-terminal coding sequences beginning at plasminogen residue 458 and expending through residue 461 of SEQ m NO:1 was synthesized:
TGGGTACCGACGACGACGACAAGTCCGAAGAAGACTGTATGTTTGGG (**SEQ ID NO:4**). A 5' PCR primer coding for a Tobacco Etch Virus (TEV) protease cleavage site Glu-Asn-Leu-Tyr-Phe-Gln (**SEQ ID NO:5**) followed by the K5 N-terminal coding sequences beginning at plasminogen residue 458 and extending through residue 461 of SEQ ID NO: 1 was synthesized:
TGGGTACCGAA.AACCTGTATTTTCAGTCCGAAGAAGACTGTATGTTTGGG (**SEQ ID NO: 6**). A K5 C-terminal 3' primer TTATTAGGCCGCACACTGAGGGA (**SEQ ID NO:7**) also was synthesized. PCR fragments were generated separately using 50 µL of Pfu DNA polymerase buffer (Stratagene^{®}, La Jolla, CA), 200 µM each dNTP, 500µM ATP, 0.5 µM each of one of the appropriate 5' primers and the 3' primer and T4 polynucleotide kinase. After incubation at 37 °C for 15 mins to kinase the primers, Pfu DNA polymerase and approximately 10 ng of DraI-digested vector pHIL-D8 containing K4-K5A plasmid (described previously in U.S. Patent No. 6,057,122) was added. Using SEQ ID NO:4 and SEQ ID NO:7 as primers, after 1 minute at 94°C, PCR was run with a thermocycle file using 20 cycles of [94°C 1 sec, 40 sec; 72°C 1 sec; 50°C 1 min, 30 sec; 72°C 1 sec, 4 mins.] which resulted in a product that encodes Ek-SEED-K5. Using SEQ ID NOs:3 and 7 and SEQ ID NOs:6 and 7 as primer sets, after 1 minute at 94°C, PCR was run with a thermocycle file using 20 cycles of [94°C 1 sec, 40 sec; 72°C 1 sec; 50°C 2 min, 30 sec; 72°C 1 sec, 4 mins.] which resulted in PCR products that encodes Ek-SLLPD-K5 and Tev-SEED-K5, respectively. The PCR products were then purified over S400-HR spin columns (Amersham Pharmacia Biotech, Piscataway, NJ).

A pET32-Kan expression vector was constructed in the following manner: pET32a DNA (Novagen; Madison, WI) was cut with Bpu 1102I + SapI (New England Biolabs; Beverly, MA) and the 3426 bp fragment (map positions 81 - 3507) was purified by agarose gel electrophoresis. pET24d DNA (Novagen, Madison, WI) was also cut with Bpu1102I + SapI and the 2341 bp. fragment (map positions 3047 - 81) containing the Kan' gene was purified by agarose gel electrophoresis. The two fragments were ligated using a rapid ligation kit (Roche Molecular Biochemicals; Indianapolis, IN) and E. coli BL21(DE3) competent cells (Novagen; Madison, WI) were transformed with the ligation mix and plated onto LB-Kan agar plates (Micro Diagnostics; Lombard, IL). Individual colonies were grown up in LB-Kan medium and plasmid DNA purified using midiprep kits (Quiagen; Valencia, CA). The DNA sequence of the coding region including the T7 promoter and T7 terminator was verified by DNA sequence analysis.

One µg of pET32-Kan was digested with BgIII plus XhoI, then the ends were filled and the DNA phosphatased and purified as described previously (U.S Patent No. 6,057,122, *supra*). One µL (approximately 10 ng) of this pET32-Kan vector and 1 µL of a K5 PCR product were ligated in a volume of 5.25 µL using a Rapid Ligation Kit as described previously. One µL of each ligation mixture was then transformed into 20 µL of HMS174(DE3) competent cells (Novagen, Madison, WI). Recombinant cells were selected on LB-Kanamycin agar. Colonies were screened by PCR for the presence of an insert of the correct size and orientation as described previously (U.S **Patent** No. 6,057,122, supra). Correct colonies were then grown up for isolation of plasmid DNA for DNA sequence verification, and for small-scale expression studies as described previously (U.S Patent No. 6,057,122, *supra*).

### Example 2

### Construction of pET32-Kan-Tev-SLLPD-K5 and pET32-Kan-Tev-LLPD-K5 (comparative)

Two 5' PCR primers coding for a TEV protease cleavage site Glu-Asn-Leu-Tyr-Pho-GIn **(SEQ ID NO:5),** one with and one without a Ser residue, followed by a K5 N-terminal coding sequences beginning at plasminogen residue 450 and extending through residue 453 of SEQ ID NO:1 were synthesized:
TGGGTACCGAAAACCTGTATTTTCAGTCCCTGCTTCCAGATGTAGAGA **(SEQ ID NO:8)** and TGGGTACCGAAAACCTGTATTTTCAGCTGCTTCCAGATGTAGAGACTC **(SEQ ID NO:9).** The C-terminal 3' primer was prepared as described in Example 1 above. PCR reactions were prepared on ice in thin-walled tubes, then transferred directly to a Perkin-Elmer 480 thermocycler block preheated to 94°C. Using SEQ ID NOs:8 and 7 and SEQ ID NOs:9 and 7 as primer sets, after 1 minute at 94°C, PCRs were run with a thermocycle file using 25 cycles of [94°C 1 sec, 40 sec; 80°C 1 sec; 45°C 5 min, 30 sec; 72°C 1 sec, 8 mins.], resulting in products encoding Tev-SLLPD-K5 and Tev-LLPD-K5, respectively. Cloning was performed as described in Example 1 above.

### Example 3

### Isolation and Purification of K5 from Fusion Proteins

(a) Isolation of Fusion Protein: Cell paste of Ek-SLLPD-K5, Ek-SEED-K5, Tev-SLLPD-K5, Tev-SEED-K5 or Tev-LLPD-K5 from Examples 1 and 2 was removed from -80°C storage and mixed with lysis buffer (50mM Tris, 300mM NaCl, 1mM NaN₃, pH 7.9, Hampton Research, Laguna Niguel, California), protease inhibitors (87 ug/mL PMSF, 5ug/mL aprotinin, 78 ug/mL benzamidine, 1ug/mL leupeptin, 5 ug/mL phenanthrolin) (SIGMA, St. Louis, Missouri), and Benzonase (20 µL/100 grams cell paste) (EM Industries, Hawthorne, New York). The cells in the suspension were lysed using a Microfluidizer at 11.000 psi (Microfluidics, Newton, Massachusetts). Typical lysis was >90% as confirmed by microscopy. The supcnsion was clarified by centrifugation (RCF = 22000g for 30 minutes at 4°C). The supernatant was decanted and mixed with Ni IMAC resin (Probond, Invitrogen Corporation, Carlsbad, California) in a batch method. The resin was washed with lysis buffer to remove non-specifically bound proteins. The fusion protein was eluted in a stepwise gradient of imidazole (50mM, 100mM, 250mM, and 500mM) in lysis buffer. The pure fusion protein was concentrated and the buffer was exchanged by dialysis to cleavage buffer (50mM Tris, 1 mM NaN3, pH 7.9) (Hampton Research, Laguna Niguel, California).
(b) Cleavage of Fusion Protein using Enterokinase: The Ek fusion protein solution was made to a final concentration of 1mM CaCl₂. Enterokinase (EKMax, Invitrogen Corporation, Carlsbad, California) was added to the fusion protein at 0.8163 to 14.3 units per gram of fusion protein as determined by absorbance at 280nm. The solution was brought to 37°C, and the cleavage reaction was allowed to continue for 20 to 24 hours. The progression of cleavage was monitored by SDS-PAGE under reducing conditions.
(c) Cleavage of Fusion Protein using REV protease: The Tev fusion protein solution was made to a final concentration of 1mM DTT (SIGMA, St. Louis, Missouri). Recombinant TEV Protease, (GibcoBRL Life Technologies, Gaithersburg, Maryland) was added to the fusion protein at 76.3 to 1330 units per gram of fusion protein as determined by absorbance at 280nm. The solution was brought to 30°C, and the cleavage reaction was allowed to continue for 20 to 24 hours. The progression of cleavage was monitored by SDS-PAGE under reducing conditions.
(d) Isolation of K5: Cleaved K5 was isolated by mixing the cleavage solution with Ni IMAC resin (Probond, Invitrogen Corporation, Carlsbad, California) in a batch method. The cleavage flow-through was collected and the resin was washed with cleavage buffer. The wash solutions that contained the K5 were combined with the cleavage flow-through, and applied to an anion exchange resin (Q Sepharose fast flow, SIGMA, St. Louis, Missouri). Pure K5 was isolated using a linear NaCl gradient (0 mM to 300mM) in cleavage buffer.

Cleavage of the fusion proteins with the appropriate enzyme resulted in products referred to hereinafter as SLLPD-K5, SEED-K5, and recombinant LLPD-K5.

### Example 4

### Functionalization of the N-Terminus of SLLPD-K5 and Conjugation with Methoxypolyethyleneglycol (mPEG. mol. wt. 20.000)

Kringle 5 peptide fragment SLLPD-K5 was functionalized and conjugated with methoxypolyethylene glycol (5-30kD) essentially as described by Gaertner et al. in Bioconjugate Chem., 7, 38-44 (1996). The protocol used is described briefly below.
(a) Formation of N-hydroxysuccinimide ester of BOC-AOA: A solution of BOC-aminooxyacetic acid (BOC-AOA, 1.9 g, purchased from Novabiochem) in ethyl acetate (30 mL) at room temperature was treated with a solution of N-hydroxysuccinimide (1.15 g) in ethyl acetate (30 mL). The resulting mixture was treated with a solution of N,N'-dicyclohexylcarbodiimide (2.06 g) in ethyl acetate (5 mL), stirred for 18 hours, filtered, and concentrated. The concentrate was triturated with diethyl ether (40 mL), filtered, and the resulting solid dried under vacuum to provide 1.2 g of the desired product.
   MS *m*/*e* 306 (M+NH₄)⁺;
   ¹H NMR δ 1.48 (9(H), 2.88 (4H), 4.78 (2H).
(b) Formation of MeO-PEG_{20kd}-NH-COCH₂-O-NH(AOA-PEG_{20kd}): A solution of MeO-PEG_{20kd}-NH₂ (1.5 g, purchased from Rapp Polymere Inc., Tubingen, Germany) in acetonitrile (4.5 mL) at room temperature was treated with Example 3A (50 mg) and 4-methylmorpholine (0.1 mL), stirred for 2 hours, and concentrated. The concentrate was dissolved in water (9 mL),treated with β-alanine (100 mg), and adjusted to pH 9.2 with 2N NaOH. The mixture was dialyzed against water (2 changes of 4 liters) using 3,500 mol. wt. cutoff dialysis tubing, then lyophilized to provide 1.4 g of BOC-AOA-PEG-OMe.
   A solution of BOC-AOA-PEG-OMe (1.4 g) in trifluoroacetic acid (TFA, 2.5 mL) at room temperature was allowed to stand for 3 hours, concentrated, treated with water (5 mL), and concentrated again. The concentrate was dissolved in water (7.5 mL) and adjusted to pH 3.0 with 2M NaOH. The solution was then dialyzed (3.5 kD cutoff dialysis tubing) against water at 4 °C and lyophilized. A portion of the resulting solid (29 mg) was dissolved in water (1 mL). The amino-oxy functional group of this solution was estimated spectrophotometrically by condensation with m-nitrobenzaldehyde (MNBA) in 50mM acetate buffer, pH 4.0. Quantitative reaction from a solution of a weighed amount of MNBA with excess AOA-PEG-OMe showed that the molar increase in absorbance of this aldehyde when converted to the mPEG-oxime is 12,400 at 255 nm. Complete reaction of AOA-PEG-OMe was accomplished in 15 minutes at 60°C when the aldehyde was 1.0 mM, and in 1.5-2 fold excess over the concentration of AOA-PEG-OMe as estimated by weight of the latter. The 29 mg/mL solution was found to be 0.95 mM (68% active), equivalent to 19 mg/mL aldehyde-reactive polymer, based on a mol wt. of 20,000.
(c) Oxidation of SLLPD-K5: Purified SLLPD-K5 at approximately 1 mg/mL was supplied in Tris-Cl buffer containing 100 mM NaCl, pH 7.8. The protein concentrations of K5, SLLPD-K5, and PEG conjugates of K5 were estimated from optical density at 280 nM, where a 1.0 mg/mL solution (1.0 cm path length, pH range 4-8.5) has OD = 1.70.
   SLLPD-K5 was dialyzed into 0.1M NH₄CO₃ (pH 7.5-8), then concentrated by filtration through 3 kd cutoff membrane to 3.3 mg/mL. The solution (66.6 mL, 220 mg kringle 5 mol. wt. 10,500) was treated with 1-methionine (50 mg) to provide 5 mM final methionine concentration. The fixture was adjusted to pH 8.6 with concentrated NH₄OH, treated with sodium periodate (18 mg, 4 equivalents), and monitored by reverse phase HPLC (C-8 Waters Symmetry 4.6 x 250 mm, acetonitrile in water with 0.1% TFA, CH₃CN increasing from 5%-40% over 30 minutes.). After 1 hour the mixture was treated with an additional 13.5 mg, stirred for an additional hour, treated with 25µL of propanediol, stirred for 1 hour, and adjusted to pH 4.8 with 1:1 glacial acetic acid/water. The mixture was dialyzed (3,500 mol wt cutoff membrane) overnight against 50 mM sodium acetate buffer, pH 5.0 (4 L) and analyzed by mass spectrum analysis, which indicated the presence of glyoxylyl kringle 5 (10,516) and its hydrate (10,534).
(d) Condensation of AOA-PEG with Glyoxylyl-LLPD-K5: A solution of Example 4(c) (3 mg/mL, 66.7 mL, 19 µmmol) was adjusted to pH 4.0 by dropwise addition of 1:1 glacial acetic acid/water. The solution was treated with 0.95 mM of Example 4(b) (34 mL, 32 µmmol, 1.68-fold excess over protein) and concentrated to 1/5 of the original volume using a stirred cell with 3,500 mol. wt. cutoff membrane, over a 4 hour period to provide a final protein concentration of approximately 10 mg/mL. The reaction was continued for 24 hours, quenched with 100 mM m-nitrobenzaldehyde in ethanol (0.12 mL), stirred for 30 minutes, and adjusted to pH 7.8 with 2M Tris base to provide the desired product.
   mPEG also was conjugated to SEED-K5 essentially as described above. The SLLPD-K5 and SEED-K5 peptides conjugated to methoxypolyethylene glycol are referred to hereinafter as mPEG-SLLPD-K5 and mPEG-SEED-K5.

### Comparative Example 5

### Generation of PEGylated Kringle Peptide Fragments

Using methods well known in the art (such as, for example, recombinant and synthetic techniques) for generating kringle peptide fragments and/or kringle peptide fragments having a naturally occuring or non-naturally occurring N-terminal serine and/or by following Scheme 1 and the examples above, the following compounds can be prepared:
(a) Kringle 1 (of plasminogen)-methoxypolyethylene glycol conjugate,
(b) Kringles 4-5 (of plasminogen)-methoxypolyethylene glycol conjugate, and
(f) Prothrombin Kringle 2-methoxypolyethylene glycol conjugate.

In addition, conjugates of each of the above-mentioned kringle peptide fragments with other polyalkylene glycols can be synthesized by substituting alternative polyalkylene glycols for methoxypolyethylene glycol in the procedures outlined in Scheme 1 and Examples 1-5.

### Example 6

### Endothelial Cell Migration Assay

A primary line of HMVEC (human dermal microvascular endothelial cells) were grown to 80-90% confluency, the cells were washed with phosphate-buffered saline (PBS), then left for 18 hours in minimal media (Eagles balanced salt solution, no supplements) containing 10% fetal calf serum (FCS). The cells were washed with PBS and trypsinized and resuspended at 2-5 x 10⁶ cells/mL, then loaded with fluorophore by incubation with Calcein AM^{™} (MOLECULAR PROBES, Eugene, Oregon) in the dark. Vascular endothelial growth factor (VEGF) in media was placed in wells of 96 well plates, and a filter then placed above these solutions. After centrifugation, re-suspension, and assessment of the extent of labeling, the cells were added to the tops of these wells with varied amounts of rK5 (amino acid position 450 to amino acid 543 of SEQ ID NO:1), purified mPEG-SLLPD-K5 or purified mPEG-SEED-K5. After 4 hours at 37°C, free cells (unmigrated) were wiped from the top filter, and fluorescence measured. Data were derived as a fraction of the number of cells which migrated in control wells containing no inhibitor and are shown in Table 1 as % inhibition. Sem represents standard error of the mean.

**Table 1: Inhibition of EC Migration by mPEG Kringle 5 Constructs**

| Protein Concentration (ng/mL) | rK5 (%I) | sem | mPEG -SLLPD-K5 (%I) | sem | mPEG-SEED-K (%I) | sem |
|---|---|---|---|---|---|---|
| 10 | 73 | 1 | 70 | 15 | 75 | 13 |
| 1 | 47 | 7 | 59 | 10 | 71 | 3 |
| 0.1 | 32 | 12 | 53 | 23 | 65 | 9 |
| 0.01 | 45 | 8 | 68 | 4 | 39 | 15 |
| 0.001 | 19 | 18 | 19 | 12 | 53 | 7 |
| 0.0001 | 8 | 8 | 33 | 21 | 46 | 9 |

As Table 1 shows, mPEG-SLLPD-K5 and mPEG-SEED-K5 inhibited endothethial cell migration to the same, if not to a greater extent, than unpegylated rK5.

### Example 7

### Monkey Pharmacokinetics

All samples were diluted in phosphate buffered saline to a concentration of 1.1 mg/mL. Nine cynomolgus monkeys, weighing 3-6 kg, were fasted overnight prior to dosing but were allowed water *ad libitum.* Food was returned to the monkeys approximately three hours after dosing. During the study the animals were housed individually.

This study was performed in parallel, in three groups of monkeys; each group contained three animals. All animals received a 1 mg/kg (0.91 mL/kg) intravenous dose of compound (either mPEG-SLLPD-K5, mPEG-SEED-K5 or recombinant LLPD-K5) administered as a slow bolus in a saphenous vein. EDTA preserved blood samples (about 3 mL) were withdrawn from a femoral artery or vein of each animal prior to dosing and at 0.1, 0.25, 0.5, 1, 2, 3, 4, 6, 9 and 24 hours after drug administration. The results are shown in Table 2 below and in Figure 2.

**Table 2**

| | 1 mg/kg IV Dose | | | | |
|---|---|---|---|---|---|
| | t½* | V_{c}⁺ | V_{β}^{^} | AUC_{0-∞}^{#} | Clₚ** |
| Form | (hr) | (L/kg) | (L/kg) | (µg•hr/mL) | (L/hr•kg) |
| r-LLPD-K5 | 0.19 | 0.033 | 0.033 | 9.85 | 0.11 |
| mPEG-SEED-K5 | 5.05 | 0.044 | 0.057 | 150.20 | 0.0078 |
| mPEG-SLLPD-K5 | 5.53 | 0.031 | 0.067 | 126.66 | 0.0081 |

| | | | | | |
|---|---|---|---|---|---|
| *t½= half-life in circulation +Vc = volume parameter ^{^}V_{β} = volume parameter ^{#}AUC_{0-∞} = area under the curve **Clₚ = clearance rate | | | | | |

As Table 2 shows, both mPEG-SEED-K5 and mPEG-SLLPD-K5 displayed a 27x longer half-life (t½) than recombinant LLPD-K5 and a 13x slower clearance rate (Clp) than non-pegylated K5 when dosed intravenously in monkeys.

### Example 8

### Conjugation of the N-Terminus of SLLPD-K5 with Methoxypolyethyleneglycol aldehyde (PEG-aldehyde, mol. wt. 20.000)

Kringle 5 peptide fragment SLLPD-K5 was functionalized and conjugated with methoxypolyethylene glycol (5-30kD) essentially as described in J. Pharmaceut. Sci., 87, 1446-1449 (1998). The protocol used is described briefly below.
(a) Titration of Methoxypolyethyleneglycol aldehyde: A mixture of 2mM methoxypolyethyleneglycol aldehyde (200 µL of 40 mg/mL in H₂O) was treated with 10mM p-nitrophenylhydrazine (200 µL, 15.3 mg in 10 mL ethanol) and incubated at room temperature for 24 hours. A 50 µL aliquot was diluted to 1.0 mL with 100mM HCl and measured for absorbance at 405 nm at t = 0 and t = 24 hrs. The change in absorbance (λ₄₀₅ = 0.64, t = 24 hrs) was compared to that of a 50uM standard of the p-nitrophenylhydrazone of 3-phenylpropanal (λ₄₀₅ = 0.67) indicating 96% aldehyde content compared to that predicted by weight.
(b) Condensation of Methoxypolyethyleneglycol aldehyde and SLLPD-K5: A mixture of SLLPD-K5 (300 mg) in 50mM sodium acetate (21.7 mL, pH 4.7) was treated with methoxypolyethyleneglycol aldehyde (1.14 g, 0.0572 mmol, purchased from Shearwater) and 5M sodium cyanoborohydride in 1M NaOH (43.4 µL, 0.217 mmol). The mixture was incubated at room temperature for 48 hours while the reaction's progress was monitored by HPLC (Waters C8 Symmetry column, 4.6 x 150 mm; solvent gradient: 5% to 40% acetonitrile/H₂O containing 0.1% TFA in 30 minutes). The mixture was treated with additional methoxypolyethyleneglycol aldehyde (200 mg) and sodium cyanoborohydride (43.4 µL), and incubated for an additional 24 hours at room temperature.
(c) Purification of mPEGylated-K5 Formed by Reductive Amination: mPEGylated-K5 was stored at 3-7 mg/mL in 50mM Tris buffer, 100mM NaCl, and 1mM NaN₃ at pH 7.7. Pegylated K5 reaction mixtures were concentrated to approximately 10 mg/mL and dialysed into a buffer containing 25 mM sodium acetate at pH 5.0 (buffer A) for chromatography. Chromatography was performed on a Tosoh Biosep (Montgomeryville, PA) TSK-SP5PW column using 25 mM sodium acetate buffer at pH 5.0. A gradient to buffer A containing 200mM NaCl at pH 5.0 (buffer B) was generated using a Pharmacia FPLC system to elute the protein. Different sized columns were used to handle different sample loads, but the most commonly used was TSK-SP5PW-HR (2 x 15 cm). Flow rates were adjusted to match column sizes and varied from 1.0 to 2.5 mL/min. Columns were equilibrated in buffer A and the sample, previously dialyzed into buffer A, was loaded. The column was then washed with up to three column volumes of the buffer A to remove excess mPEG-aldehyde, its removal followed by absorbance at 280 nm. After column washing, a gradient was initiated over 2 to 3 column volumes from buffer A to 25% buffer B (50mM NaCl). The gradient was put on hold at 50mM NaCl until the absorbance was restored to baseline. At that point the gradient was increased to 100% buffer B (200mM NaCl) to elute unreacted K5. Three peaks ehrted in the 50mM NaCl "hold gradient" stage. SDS Polyacrylamide gel electrophoresis (SDS-PAGE) analysis indicated the first (minor) peak to be about 90,000 MW (presumably di-mPEGylated product). A second minor peak eluted at about 50,000 Da and separated from the third (major) peak also traveling around 50,000 Da on the gel. Fractions from the major peak were pooled by SDS PAGE. The major peak (80-90% of the total) was further characterized for purity and identity by the methods below and was judged to be the N-terminal mono-mPEGylated K5.
Methods used to characterize N-terminal mono-mPEGylated K5:
1. Mass Spectrometry;
2. Analytical Chromatography on Mono-S (HR);
3. Analytical Chromatography on Mono-Q (HR);
4. Hydrophobic Interaction on TSK Phenyl 5PW;
5. Gel Filtration on Superdex 75 (HR);
6. Cyanogen Bromide Cleavage;
7. N-Terminal Sequencing; and
8. Amino Acid Composition Analysis.

### Comparative Example 9

### Generation of mPEGylated Kringle Peptide Fragments

Using methods well known in the art (such as, for example, recombinant and synthetic techniques) for generating kringle peptide fragments and/or kringle peptide fragments having a naturally occurring or non-naturally occurring N-terminal serine and/or by following Scheme 2 and the example above, the following compounds can be prepared:
(a) Kringle 1 (ofplasminogen)-methoxypolyethylene glycol conjugate
(e) Kringles 4-5 (of plasminogen)-methoxypolyethylene glycol conjugate, and
(f) Prothrombin Kringle 2-methoxypolyethylene glycol conjugate.
In addition, conjugates of each of the above-mentioned kringle peptide fragments with other polyalkylene glycols can be synthesized by substituting alternative polyalkylene glycols for methoxypolyethylene glycol in the procedures outlined in Scheme 2 and Example 8.

### Example 10

### Monkey Pharmacokinetics

Intravenous dosing was done in cynomolgus monkeys at 1 mg/kg (n= 3). Plasma concentrations were determined by Western blot analysis. The results are shown in Table 3.

**Table 3**

| | Plasma Conc. (µg/ml) | | |
|---|---|---|---|
| Time | rK5 | 20 Kd | 20 Kd |
| (hours) | (sem) | Oxime PEG K5 | Reductive |
| | | (sem) | Amination |
| | | | mPEGK5 (sem) |
| 0.1 | 20.6 (1.44) | 30.2 (1.13) | 30.1(2.62) |
| 0.25 | 14.34 (1.97) | 27.4 (1.12) | 32.0 (1.84) |
| 0.5 | 6.38 | 26.3 (1.2) | 33.64 (1.24) |
| 1.0 | 3.47 | 22.12 (2.66) | 31.0(0.68) |
| 2 | 0.79 | 17.23 (0.82) | 29.64 (0.93) |
| 3.0 | - | 10.89 (0.25) | 23.93 (0.37) |
| 4.0 | - | 9.26 (2.1) | 20.84 (0.47) |
| 6.0 | - | 5.98 (1.3) | 16.1 (1.85) |
| 9.0 | ND | 2.29 (0.1) | 11.57 (1.96) |
| 24.0 | ND | 0.7 (0.26) | 1.69 (0.46) |
| 36.0 | ND | ND | 0.73 (0.15) |

| | | | |
|---|---|---|---|
| sem = standard error of the mean ND = not done | | | |

As Table 3 shows, mPEGylation at the N-terminus with 20 kD mPEG dramatically decreases the clearance from plasma in monkeys as compared to non-mPEGylated K5. mPEGylation accomplished via reductive amination results in slower clearance of the peptide as compared to mPEGylation by oxime formation.

## Claims

1. A conjugated kringle peptide fragment selected from the group consisting of mPEG-SLLPD-K5 and mPEG-SEED-K5.

2. A pharmaceutical composition comprising a conjugated kringle peptide fragment of claim 1 in combination with a therapeutically acceptable carrier.

3. A conjugated kringle peptide fragment of claim 1 for treating a disease in a patient in need of anti-angiogenic therapy by administration to a human or animal of a therapeutically effective amount of said conjugated kringle peptide fragment.

4. The conjugated kringle peptide fragment of claim 3 wherein the disease is selected from the group consisting of cancer, arthritis, macular degeneration, and diabetic retinopathy.

5. The conjugated kringle peptide fragment of claim 4 wherein the disease is cancer.

6. The conjugated kringle peptide fragment of claim 5 wherein the disease is selected from primary and metastatic solid tumors, carcinomas, sarcomas, lymphomas, psoriasis, and hemagiomas.

7. A conjugated kringle peptide fragment of claim 1 for inhibiting endothelial cell proliferation in an individual by administration to said individual of an effective amount of said conjugated kringle peptide fragment.

## Patentansprüche

1. Ein konjugiertes Kringle Peptidfragment gewählt aus der Gruppe bestehend aus mPEG-SLLPD-K5 und mPEG-SEED-K5.

2. Eine pharmazeutische Zusammensetzung, die ein konjugiertes Kringle Peptidfragment von Anspruch 1 in Kombination mit einem therapeutisch verträglichen Träger umfaßt.

3. Ein konjugiertes Kringle Peptidfragment von Anspruch 1, zur Behandlung einer Krankheit in einem Patienten, der eine antiangiogene Therapie benötigt, durch Verabreichen einer therapeutisch wirksamen Menge des konjugierten Kringle Peptidfragments an einen Menschen oder ein Tier.

4. Das konjugierte Kringle Peptidfragment von Anspruch 3, worin die Krankheit gewählt ist aus der Gruppe bestehend aus Krebs, arthritis, Makuladegeneration und diabetischer Retinopathie.

5. Das konjugierte Kringle Peptidfragment von Anspruch 4, worin die Krankheit Krebs ist.

6. Das konjugierte Kringle Peptidfragment von Anspruch 5, worin die Krankheit gewählt ist aus primären und metastatischen festen Tumoren, Karzinomen, Särkomen, Lymphomen, Psoriasis und Hämangiomen.

7. Ein konjugiertes Kringle Peptidfragment von Anspruch 1 zur Hemmung der Endothelialzellproliferation in einem Individuum durch Verabreichen einer wirksamen Menge des konjugierten Kringle Peptidfragments an das Individuum.

## Revendications

1. Fragment de peptide kringle conjugué choisi dans le groupe consistant en mPEG-SLLPD-K5 et mPEG-SEED-K5.

2. Composition pharmaceutique comprenant un fragment de peptide kringle conjugué selon la revendication 1 en combinaison avec un véhicule thérapeutiquement acceptable.

3. Fragment de peptide kringle conjugué selon la revendication 1 pour traiter une maladie chez un patient ayant besoin d'une thérapie anti-angiogène par administration à un humain ou à un animal d'une quantité thérapeutiquement efficace dudit fragment de peptide kringle conjugué.

4. Fragment de peptide kringle conjugué selon la revendication 3 dans lequel la maladie est choisie dans le groupe consistant en le cancer, l'arthrite, la dégénérescence maculaire et la rétinopathie diabétique.

5. Fragment de peptide kringle conjugué selon la revendication 4 dans lequel la maladie est le cancer.

6. Fragment de peptide kringle conjugué selon la revendication 5 dans lequel la maladie est choisie parmi les tumeurs solides primitives ou métastasiqucs, les carcinomes, les sarcomes, les lymphomes, le psoriasis et les hémangiomes.

7. Fragment de peptide kringle conjugué selon la revendication 1 pour inhiber la prolifération des cellules endothéliales chez un sujet par administration audit sujet d'une quantité efficace dudit fragment de peptide kringle conjugué.


### SEQUENCE LISTING

<110> Abbott Laboratories
   Henkin, Jack
   Davidson, Donald J.
<120> ANTIANGIOGENIC POLYPEPTIDES AND METHODS FOR INHIBITING ANGIOGENESIS
<130> 6738.PC.01
<140> Not Yet Assigned
   <141> 2001-09-28
<150> Not Yet Assigned
   <151> 2001-08-31
<150> US 09/675,226
   <151> 2000-09-29
<160> 7
<170> FastSEQ for Windows Version 4.0
<210> 1
   <211> 791
   <212> PRT
   <213> Homo sapiens
<400> 1
   Glu Pro Leu Asp Asp Tyr Val Asn Thr Gln Gly Ala Ser Leu Phe Ser
    1               5                  10                  15
   Val Thr Lys Lys Gln Leu Gly Ala Gly Ser Ile Glu Glu Cys Ala Ala
               20                  25                  30
   Lys Cys Glu Glu Asp Glu Glu Phe Thr Cys Arg Ala Phe Gln Tyr His
           35                  40                  45
   Ser Lys Glu Gln Gln Cys Val Ile Met Ala Glu Asn Arg Lys Ser Ser
       50                  55                  60
   Ile Ile Ile Arg Met Arg Asp Val Val Leu Phe Glu Lys Lys Val Tyr
   65                  70                  75                  80
   Leu Ser Glu Cys Lys Thr Gly Asn Gly Lys Asn Tyr Arg Gly Thr Met
                   85                  90                  95
   Ser Lys Thr Lys Asn Gly Ile Thr Cys Gln Lys Trp Ser Ser Thr Ser
               100                 105                 110
   Pro His Arg Pro Arg Phe Ser Pro Ala Thr His Pro Ser Glu Gly Leu
           115                 120                 125
   Glu Glu Asn Tyr Cys Arg Asn Pro Asp Asn Asp Pro Gln Gly Pro Trp
       130                 135                 140
   Cys Tyr Thr Thr Asp Pro Glu Lys Arg Tyr Asp Tyr Cys Asp Ile Leu
   145                 150                 155                 160
   Glu Cys Glu Glu Glu Cys Met His Cys Ser Gly Glu Asn Tyr Asp Gly
                   165                 170                 175
   Lys Ile Ser Lys Thr Met Ser Gly Leu Glu Cys Gln Ala Trp Asp Ser
               180                 185                 190
   Gln Ser Pro His Ala His Gly Tyr Ile Pro Ser Lys Phe Pro Asn Lys
               195                     200                     205
   Asn Leu Lys Lys Asn Tyr Cys Arg Asn Pro Asp Arg Glu Leu Arg Pro
       210                     215                     220
   Trp Cys Phe Thr Thr Asp Pro Asn Lys Arg Trp Glu Leu Cys Asp Ile
   225                     230                     235                 240
   Pro Arg Cys Thr Thr Pro Pro Pro Ser Ser Gly Pro Thr Tyr Gln Cys
                   245                     250                     255
   Leu Lys Gly Thr Gly Glu Asn Tyr Arg Gly Asn Val Ala Val Thr Val
               260                     265                     270
   Ser Gly His Thr Cys Gln His Trp Ser Ala Gln Thr Pro His Thr His
           275                     280                     285
   Asn Arg Thr Pro Glu Asn Phe Pro Cys Lys Asn Leu Asp Glu Asn Tyr
       290                     295                     300
   Cys Arg Asn Pro Asp Gly Lys Arg Ala Pro Trp Cys His Thr Thr Asn
   305                     310                     315                 320
   Ser Gln Val Arg Trp Glu Tyr Cys Lys Ile Pro Ser Cys Asp Ser Ser
                   325                     330                     335
   Pro Val Ser Thr Glu Gln Leu Ala Pro Thr Ala Pro Pro Glu Leu Thr
               340                     345                     350
   Pro Val Val Gln Asp Cys Tyr His Gly Asp Gly Gln Ser Tyr Arg Gly
           355                     360                     365
   Thr Ser Ser Thr Thr Thr Thr Gly Lys Lys Cys Gln Ser Trp Ser Ser
       370                     375                     380
   Met Thr Pro His Arg His Gln Lys Thr Pro Glu Asn Tyr Pro Asn Ala
   385                     390                     395                 400
   Gly Leu Thr Met Asn Tyr Cys Arg Asn Pro Asp Ala Asp Lys Gly Pro
                   405                     410                     415
   Trp Cys Phe Thr Thr Asp Pro Ser Val Arg Trp Glu Tyr Cys Asn Leu
               420                     425                     430
   Lys Lys Cys Ser Gly Thr Glu Ala Ser Val Val Ala Pro Pro Pro Val
           435                     440                     445
   Val Leu Leu Pro Asp Val Glu Thr Pro Ser Glu Glu Asp Cys Met Phe
       450                     455                     460
   Gly Asn Gly Lys Gly Tyr Arg Gly Lys Arg Ala Thr Thr Val Thr Gly
   465                     470                     475                 480
   Thr Pro Cys Gln Asp Trp Ala Ala Gln Glu Pro His Arg His Ser Ile
                   485                     490                     495
   Phe Thr Pro Glu Thr Asn Pro Arg Ala Gly Leu Glu Lys Asn Tyr Cys
               500                     505                     510
   Arg Asn Pro Asp Gly Asp Val Gly Gly Pro Trp Cys Tyr Thr Thr Asn
           515                     520                     525
   Pro Arg Lys Leu Tyr Asp Tyr Cys Asp Val Pro Gln Cys Ala Ala Pro
       530                     535                     540
   Ser Phe Asp Cys Gly Lys Pro Gln Val Glu Pro Lys Lys Cys Pro Gly
   545                     550                     555                 560
   Arg Val Val Gly Gly Cys Val Ala His Pro His Ser Trp Pro Trp Gln
                   565                     570                     575
   Val Ser Leu Arg Thr Arg Phe Gly Met His Phe Cys Gly Gly Thr Leu
               580                     585                     590
   Ile Ser Pro Glu Trp Val Leu Thr Ala Ala His Cys Leu Glu Lys Ser
           595                     600                     605
   Pro Arg Pro Ser Ser Tyr Lys Val Ile Leu Gly Ala His Gln Glu Val
       610                     615                     620
   Asn Leu Glu Pro His Val Gln Glu Ile Glu Val Ser Arg Leu Phe Leu
   625                     630                     635                 640
   Glu Pro Thr Arg Lys Asp Ile Ala Leu Leu Lys Leu Ser Ser Pro Ala
                   645                     650                     655
   Val Ile Thr Asp Lys Val Ile Pro Ala Cys Leu Pro Ser Pro Asn Tyr
               660                 665                 670
   Val Val Ala Asp Arg Thr Glu Cys Phe Ile Thr Gly Trp Gly Glu Thr
           675                 680                 685
   Gln Gly Thr Phe Gly Ala Gly Leu Leu Lys Glu Ala Gln Leu Pro Val
       690                 695                 700
   Ile Glu Asn Lys Val Cys Asn Arg Tyr Glu Phe Leu Asn Gly Arg Val
   705                 710                 715                 720
   Gln Ser Thr Glu Leu Cys Ala Gly His Leu Ala Gly Gly Thr Asp Ser
                   725                 730                 735
   Cys Gln Gly Asp Ser Gly Gly Pro Leu Val Cys Phe Glu Lys Asp Lys
               740                 745                 750
   Tyr Ile Leu Gln Gly Val Thr Ser Trp Gly Leu Gly Cys Ala Arg Pro
           755                 760                 765
   Asn Lys Pro Gly Val Tyr Val Arg Val Ser Arg Phe Val Thr Trp Ile
       770                 775                 780
   Glu Gly Val Met Arg Asn Asn
   785                 790
<210> 2
   <211> 5
   <212> PRT
   <213> Artificial Sequence
<220>
   <223> Enterokinase Cleavage Site
<400> 2
   Asp Asp Asp Asp Lys
   1               5
<210> 3
   <211> 45
   <212> DNA
   <213> Artificial Sequence
<220>
   <223> Forward Primer
<400> 3
   tgggtaccga cgacgacgac aagtccctgc ttccagatgt agaga 45
<210> 4
   <211> 47
   <212> DNA
   <213> Artificial Sequence
<220>
   <223> Forward Primer
<400> 4
   tgggtaccga cgacgacgac aagtccgaag aagactgtat gtttggg 47
<210> 5
   <211> 6
   <212> PRT
   <213> Artificial Sequence
<220>
   <223> TEV Cleavage Site
<400> 5
   Glu Asn Leu Tyr Phe Gln
    1               5
<210> 6
   <211> 23
   <212> DNA
   <213> Artificial Sequence
<220>
   <223> Reverse Primer
<400> 6
   ttattaggcc gcacactgag gga 23
<210> 7
   <211> 48
   <212> DNA
   <213> Artificial Sequence
<220>
   <223> Forward Primer
<400> 7
   tgggtaccga aaacctgtat tttcagctgc ttccagatgt agagactc 48